(19) Europäisches Patentamt
European Patent Office
Office européen des brevets

(11) Veröffentlichungsnummer : **0 372 330 B1**

# (12) EUROPÄISCHE PATENTSCHRIFT

(45) Veröffentlichungstag der Patentschrift : **03.02.93 Patentblatt 93/05**

(51) Int. Cl.$^5$ : **C07C 233/60**

(21) Anmeldenummer : **89121783.8**

(22) Anmeldetag : **25.11.89**

## (54) Speziell substituierte Cyclopropancarboxamide, Verfahren zu ihrer Herstellung und ihre Verwendung zur Bekämpfung von Schädlingen.

(30) Priorität : **09.12.88 DE 3841433**

(43) Veröffentlichungstag der Anmeldung : **13.06.90 Patentblatt 90/24**

(45) Bekanntmachung des Hinweises auf die Patenterteilung : **03.02.93 Patentblatt 93/05**

(84) Benannte Vertragsstaaten : **AT BE CH DE ES FR GB IT LI NL**

(56) Entgegenhaltungen :
**EP-A- 0 004 334**
**EP-A- 0 258 733**

(73) Patentinhaber : **BASF Aktiengesellschaft**
**Carl-Bosch-Strasse 38**
**W-6700 Ludwigshafen (DE)**

(72) Erfinder : **Kardorff, Uwe**
**D 3,4**
**W-6800 Mannheim 1 (DE)**
Erfinder : **Neubauer, Hans-Jürgen**
**Mozartstrasse 6**
**W-4400 Münster-Hiltrup (DE)**
Erfinder : **Leyendecker, Joachim**
**Stahlbühlring 79**
**W-6802 Ladenburg (DE)**
Erfinder : **Kuenast, Christoph**
**Salierstrasse 2**
**W-6701 Otterstadt (DE)**
Erfinder : **Hofmeister, Peter**
**Bernard-Humblot-Strasse 12**
**W-6730 Neustadt (DE)**
Erfinder : **Krieg, Wolfgang**
**Saarstrasse 17**
**W-6721 Weingarten (DE)**
Erfinder : **Buerstinghaus, Rainer**
**Robert-Koch-Strasse 2**
**W-4404 Telgte (DE)**

EP 0 372 330 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Cyclopropancarboxamide der allgemeinen Formel I

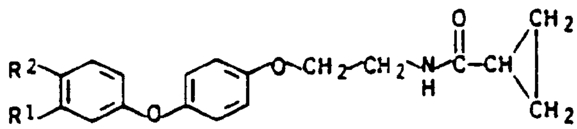 (I)

in der die Substituenten die folgende Bedeutung haben:

$R^1$ $C_1$-$C_4$-Alkyl, Fluor, Chlor oder Brom, $C_1$-$C_3$-Halogenalkyl, $C_1$-$C_3$-Halogenalkyloxy
$R^2$ Wasserstoff, Fluor, Chlor oder Brom,
mit der Maßgabe, daß $R^1$ im Fall von $R^2$ = H nicht Fluor bedeutet.

Außerdem betrifft die vorliegende Erfindung die Herstellung der Verbindungen 1, Schädlingsbekämpfungsmittel, die die Verbindungen I enthalten und ein Verfahren zur Bekämpfung von Schädlingen.

Aus der DE-A 36 28 082 sind verschieden substituierte Cyclopropancarboxamide der Struktur A

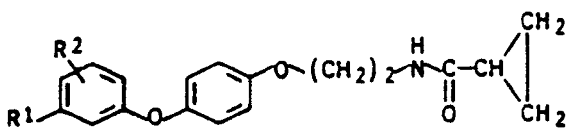 A

bekannt, in der der Rest $R^2$ bevorzugt in 4-Stellung steht, worin $R^1$ Wasserstoff bedeutet und u.a. für Halogen, $C_1$-$C_3$-Alkyl oder Trifluormethyl steht. Im Fall einer Disubstitution im 4-Phenoxyrest die für Halogensubstituenten beschrieben ist, ist die Kombination 3,5-Dihalogen bzw. 2,4-Difluor angegeben.

Der Erfindung lag die Aufgabe zugrunde, neue Cyclopropancarboxamide zur Verfügung zu stellen, die in ihrer Wirkung bekannten Cyclopropancarboxamiden überlegen sind.

Demgemäß wurde gefunden, daß die eingangs definierten speziell substituierten Cyclopropancarboxamide der Formel I sich ausgezeichnet zur Bekämpfung von Schädlingen eignen.

Im Hinblick auf die aus dem Stand der Technik bekannten Verbindungen ist überraschend, daß eine Substitution des 4-Phenoxyrestes in 3-Stellung ggf. in Kombination mit einer Halogensubstitution in 4-Stellung zu Cyclopropancarboxamiden mit besonders vorteilhaften Eigenschaften (siehe Vergleichsversuche A bis D) führt.

Die Herstellung der Cyclopropancarboxamide I erfolgt in an sich bekannter Weise aus den 2-(4-phenoxyphenoxy)-ethylaminen II

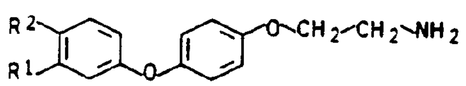 (II)

durch Umsetzung mit den entsprechenden Cyclopropancarbonsäurehalogeniden III

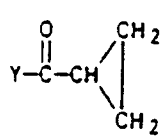 (III),

worin Y für Halogen wie Fluor, Chlor und Brom, vorzugsweise Chlor steht, in Gegenwart eines Säureakzeptors.

Als Säureakzeptor kann das 4-Phenoxyphenoxy-ethylamin II eingesetzt werden, üblicherweise verwendet man jedoch an sich übliche Basen als säurebindendes Mittel, insbesondere aliphatische aromatische oder heterocyclische Amine, beispielsweise Triethylamin, Dimethylamin, Diisopropylamin, Piperidin, Dimethylanilin, Dimethylbenzylamin, Pyridin und 4-Dimethylaminopyridin. Hydroxide von Alkali- und Erdalkalimetallen wie zum Beispiel Natriumhydroxid, Kaliumhydroxid, Calciumhydroxid; Alkoholate von Alkali-und Erdalkalimetallen wie

beispielsweise Natriummethylat, Natriumethylat, Calciummethylat, Kalium-tert.-butylat; Alkali- oder Erdalkalimetallhydride wie beispielsweise Natriumhydrid, Kaliumhydrid oder Calciumhydrid; Alkali-oder Erdalkalimetallcarbonate wie beispielsweise Natriumcarbonat, Kaliumcarbonat oder Calciumcarbonat.

Das Verhältnis Säureakzeptor zu Säurehalogenid III ist nicht besonders kritisch und liegt im allgemeinen bei 0,1 : 1 bis 20 : 1, vorzugsweise 0,7 : 1 bis 5 : 1, besonders bevorzugt 0,9 : 1 bis 1,5 : 1.

Üblicherweise setzt man die Ausgangsstoffe II und III in stöchiometrischem Verhältnis ein. Ein Überschuß der einen oder anderen Komponente kann in Einzelfällen aber durchaus vorteilhaft sein.

Die Umsetzung verläuft gewöhnlich oberhalb von -30°C mit ausreichender Geschwindigkeit. Im allgemeinen liegt die Temperatur bei -30 bis 130°, insbesondere -10 bis 80°C. Da die Umsetzung in einigen Fällen unter Wärmeentwicklung abläuft, kann es vorteilhaft sein, eine Kühlmöglichkeit vorzusehen.

Die Umsetzung wird zweckmäßigerweise in einem Lösungs- oder Verdünnungsmittel bei Normaldruck, im über- oder Unterdruck durchgeführt.

Als Lösungs- oder Verdünnungsmittel eignen sich aliphatische aromatische, gegebenenfalls chlorierte Kohlenwasserstoffe wie beipielsweise Petrolether, n-Pentan, n-Hexan, Hexan-Isomerengemische, Benzol, Toluol, Xylol, Benzin, Dichlormethan, Chloroform, Tetrachlorkohlenstoff, 1,2-Dichlorethan und Chlorbenzol; Ether und Ester wie Diethyl- und Di-n-butylether, Methyl-tert.-butylether, Tetrahydrofuran, Dioxan, Essigsäureethylester; Ketone wie Aceton, Methylethylketon, Methylisopropylketon, Nitrile wie Aceto- und Propionitril; Alkohole wie Methanol, Ethanol, n-Propanol, Isopropanol, aprotische dipolare Lösungsmittel wie Dimethylformamid, Dimethylsulfoxid, Pyridin. Auch Gemische dieser Stoffe können als Lösungs- und Verdünnungsmittel verwendet werden.

Die zur Herstellung der Verbindungen I benötigten 2-(4-Phenoxyphenoxy)-ethylamine II sind zum Teil aus Houben/Weyl, Bd. VI, 3, Methoden der organischen Chemie, Thieme Verlag, 1965, 85 ff, bekannt oder können nach den dort beschriebenen Methoden hergestellt werden.

Die außerdem benötigten Säurehalogenide III sind bekannt und insbesondere das Säurechlorid kommerziell erhältlich.

Die erfindungsgemäßen Verbindungen der Formel I können außerdem noch nach praktisch allen bekannten Verfahren der Carboxamidsynthese dargestellt werden, beispielsweise durch Umsetzung von 2-(4-Phenoxyphenoxy)-ethylaminen II mit entsprechenden Cyclopropancarbonsäureestern, Cyclopropancarbonsäuren und deren Salze, Cyclopropancarbonsäureanhydriden oder Ketonderivaten (vgl. Ferri, Reaktionen der organischen Synthese, Georg Thieme Verlag, Stuttgart, 1978, S, 542 und dort zitierte Literatur).

Die neuen Verbindungen der Formel I fallen teilweise in Form farbloser oder schwach bräunlich gefärbter Öle an, die sich durch längeres Erwärmen unter vermindertem Druck auf mäßig erhöhte Temperaturen ("Andestillieren") von den letzten flüchtigen Anteilen befreien und auf diese Weise reinigen lassen. Erhält man die verbindungen der Formel I in kristalliner Form, so kann ihre Reinigung durch Umkristallisation erfolgen.

Die Reste $R^1$ und $R^2$ in Formel I haben im einzelnen die folgende Bedeutung:

$R^1$

- unverzweigtes oder verzweigtes $C_1$-$C_4$-Alkyl wie Methyl, Ethyl, Propyl, iso-Propyl, Butyl, iso-Butyl, sec.-Butyl, tert.-Butyl
- Fluor, Chlor oder Brom
- $C_1$-$C_3$-Halogenalkyl, bevorzugt Fluor- oder Chloralkyl wie Fluormethyl, Difluormethyl, Trifluormethyl, Pentafluorethyl, 2,2,2-Trifluorethyl, Trichlormethyl und 2,2,2-Trichlorethyl, wobei der Trifluormethylrest besonders bevorzugt ist
- $C_1$-$C_3$-Halogenalkoxy, bevorzugt Fluor- oder Chloralkoxy wie Trifluormethoxy, 1,1,2,2-Tetrafluorethoxy, Pentafluorethoxy, Trichlormethoxy, wobei der Trifluormethoxyrest und der Chloralkoxyrest besonders bevorzugt sind

$R^2$

- Wasserstoff
- Fluor, Chlor oder Brom, insbesondere Fluor

Im Gegensatz zu den meisten bisher bekannten Schädlingsbekämpfungsmitteln, die als Kontakt- oder Fraßgifte die Tiere töten, lähmen oder vertreiben, greifen die Verbindungen der Formel I in das Hormonsystem des tierischen Organismus ein. Bei Insekten wird beispielsweise die Umwandlung zur Imago, die Ablage von entwicklungsfähigen Eiern und die Entwicklung von abgelegten, normalen Eiern gestört und dadurch die Generationsfolge unterbrochen. Für Wirbeltiere sind die erfindungsgemäßen Mittel praktisch ungiftig.

Die meisten Verbindungen der Formel I werden überdies leicht zu Stoffen abgebaut, die in der natürlichen Umgegebung vorkommen und von Mikroorganismen weiter zerlegt werden.

Die Cyclopropancarboxamide der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 1 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 4 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 3 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 5 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 Gew.%.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0

kg/ha.

Zu den Wirkstoffen können Öle verschiedenen Typs, Herbizide, Fungizide, andere Schädlingsbekämpfungsmittel, Bakterizide, gegebenenfalls auch erst unmittelbar Schädlingsbekämpfungsmittel, Bakterizide, gegebenenfalls auch erst können zu den erfindungsgemäßen Mitteln im Gewichtsverhältnis 1:10 bis 10:1 zugemischt werden.

Beispiel 1

N-2-[4-(3-Methylphenoxy)-phenoxy]ethyl-cyclopropancarboxamid

Zu 4,3 g (17,7 mmol) [4-(3-methylphenoxy)-phenoxy]-ethylamin in 17,5 ml Pyridin werden 2,0 g Cyclopropancarbonsäurechlorid in 5,8 ml Dichlormethan bei Raumtemperatur zugetropft und nach Beendigung des Zutropfens 14 h bei Raumtemperatur gerührt. Dann wird in Wasser gegossen und die organische Phase abgetrennt. Die Wasserphase wird mit Dichlormethan extrahiert und die vereinigten organischen Phasen mit Wasser gewaschen. Nach dem Trocknen wird im Vakuum eingeengt und der zurückbleibende Rückstand wird aus Hexan/Essigsäureethylester umkristallisiert. Man erhält eine weiße, kristalline Verbindung. Festpunkt: 114 °C
Ausbeute: 4,5 g; 82 % der rechnerisch möglichen Menge $C_{19}H_{21}NO_3$
ber.: C: 73,29%; H: 6,80 %; N: 4,50 %;
gef.: C: 72,3 %; H: 6,8 %; N: 4,5 %;
Infrarotabsorptionen [$cm^{-1}$]: 1502, 1484, 1461, 1262, 1241, 1206, 1057.

Diesem Beispiel entsprechend können analog die in der folgenden Tabelle beschriebenen Verbindungen I hergestellt werden:

Tabelle 1: Cyclopropancarboxamide I

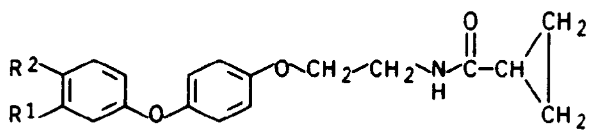

(I)

| Beispiel | $R^1$ | $R^2$ | Schmpkt. (°C) |
|---|---|---|---|
| 2 | Cl | H | 87 - 90 |
| 3 | Br | H | 88 - 90 |
| 4 | $F_3C$ | H | 67 - 68 |
| 5 | $C_2H_5$ | H | 84 |
| 6 | $OCF_3$ | H | 85 - 88 |
| 7 | Cl | F | 55 - 57 |
| 8 | F | F | 67 |

Anwendungsbeispiele

In den folgenden Beispielen wurden die erfindungsgemäßen Verbindungen bzw. sie enthaltende Mittel hinsichtlich ihrer Wirkung auf Schädlinge mit den Verbindungen der EP-A 258 733 (deutsche Anmeldung P 36 28 082) verglichen. Die Reinheit der Substanzen wie der Vergleichsmittel lag bei > 95 %.

Die Konzentrationen, bei denen die untersuchten Verbindungen eine 100 %ige bzw. 80 %ige Mortalität bzw. Hemmung bewirken, sind die jeweiligen Minimalkonzentrationen. (Wirkungsschwelle) Bei jeder Konzentration wurden mindestens zwei Versuche durchgeführt und ein Mittelwert gebildet.

Als Formulierung wurde ein 10 %iges Emulsionskonzentrat, enthaltend 70 Gew.% Cyclohexanon, 20 Gew.% Nekanil LN® (≙ Lutensol AP6, Netzmittel mit Emulgier- und Dispergierwirkung auf Basis ethoxylierter Alkylphenole), Emulphor EL® (≙ Emulan EL®, Emulgator auf Basis ethoxylierter Fettalkohole) und 10 % Wirkstoff gewählt. Die in den Beispielen angegebenen Konzentrationen wurden durch Verdünnen des formulierten Wirkstoffes mit Wasser erhalten.

Beispiel A

Dysdercus intermedius (Baumwollwanze), ovizide Wirkung

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt.

Die Eier werden für ca. 5 Sekunden in die wäßrige Wirkstoffaufbereitung getaucht, anschließend läßt man auf Filterpapier abtropfen. Dabei sollen die Eier nicht auf das Papier gelegt werden.

Die zurechtgeschnittenen Etiketten werden in Plastikpaletten gestellt, mit dem Klebestreifen nach oben. Eine halbe mit Wasser durchfeuchtete Watterolle wird in den Becher gelegt, um Austrocknung zu vermeiden und die Palette mit einer Glasplatte abgedeckt.

Nach ca. 8 Tagen wird bonitiert. (Bei der Kontrolle müssen die Larven geschlüpft sein).

Beurteilt wird die Schlupfhemmung in %. Die Versuchsergebnisse sind in Tabelle A zusammengestellt:

Tabelle A

| Verbindung Nr. | Substitution im 4-Phenoxyrest | Wirkstoffkonzentration (ppm) | Hemmung (%) |
|---|---|---|---|
| 8 | 3,4-$F_2$ | 0,4 | 80 |
| 19* | 2,4-$F_2$ | 400 | 80 |
| | | 0,4 | 0 |

* aus EP-A 258 733

Beispiel B

Dysdercus intermedius (Baumwollwanze); Zuchtversuch

Der Versuch erfolgt in 1 l-Gläser auf 200 g sterilem Quarzsand (Korngröße 0-3 mm). Dieser Sand wird vor Versuchsbeginn mit 20 ml der wäßrigen Wirkstoffaufbereitung angegossen. Darauf belegt man die Gläser mit 10 Larven des vierten Larvenstadiums. Als Futter bietet man gequollene Baumwollsaat, die wöchentlich gewechselt wird. Ebenfalls wöchentlich wird der Sand mit Wasser angefeuchtet. Die Versuchstemperatur beträgt 25 bis 27°C. Die Beobachtungszeit erstreckt sich bis zur Adulthäutung. Die Probe gilt als wirksam, wenn die Tiere bei Versuchsende entweder tot sind oder Riesenlarven oder Zwischentypen (Adultoide) darstellen oder stärkere morphologische Defekte aufweisen, also lebensunfähig sind (bezeichnet mit Mortalität in Tabelle B).

Die Versuchsergebnisse sind in Tabelle B zusammengefaßt.

Tabelle B

| Verbindung Nr. | Substitution im 4-Phenoxyrest | Wirkstoffkonzentration (ppm) | Mortalität (%) |
|---|---|---|---|
| 1 | 3-$CH_3$ | 1 | 100 |
| 22* | 4-$CH_3$ | 20 | keine Wirkung |
| 3 | 3-Br | 0,04 | 100 |
| 27* | 4-Br | 10 | keine Wirkung |

* aus EP-A 258 733

Beispiel C

Zuchtversuch mit Aedes aegypti (Gelbfiebermücken)

Bei 25°C werden in einem 250 ml Plastikbecher 200 ml Leitungswasser mit der Wirkstoffaufbereitung versetzt und anschließend mit 20 bis 30 Moskitolarven im dritten bis vierten Larvenstadium besetzt. Während der

Versuchsdauer wird einmal mit einem gepulverten, käuflichen Zierfischfutter (Tetramin®) gefüttert. Nach ca. 10 bis 12 Tagen wird die Mortalität beurteilt.

Die Versuchsergebnisse sind in Tabelle C zusammengestellt.

Tabelle C

| Verbindung Nr. | Substitution im 4-Phenoxyrest | Wirkstoffkonzentration (ppm) | Mortalität (%) |
|---|---|---|---|
| 2 | 3-Cl | 0,0002 | 100 |
| 3 | 3-Br | 0,1 | 100 |
| 17* | 3-F | 0,1 | keine Wirkung |
| 15* | 4-Cl | 0,02 | 100 |
| | | 0,0002 | keine Wirkung |

* aus EP-A 258 733

Beispiel D

Prodenia litura (ägypt. Baumwollwurm); Zuchtversuch

Die Zucht erfolgt in 100 ml Bechern auf ca. 50 ml des Standard-Nährbodens, dem im flüssigen Zustand der Wirkstoff sorgfältig untergemischt wurde. Je Konzentration wird 1 Becher mit 5 Raupen des vierten Larvenstadiums angesetzt. Die Versuchstemperatur beträgt 25 bis 26°C. Die Beobachtung erstreckt sich bis zum Schlüpfen der Falter. Die Probe gilt als wirksam, wenn Riesenlarven produziert werden.

Die Versuchsergebnisse sind in Tabelle D zusammengefaßt.

| Verbindung Nr. | Substitution im 4-Phenoxyrest | Wirkstoffkonzentration (ppm) | Mortalität (%) |
|---|---|---|---|
| 4 | 3-$CF_3$ | 0,0002 | 100 |
| 1 | 3-$CH_3$ | 0,2 | 100 |
| 22* | 4-$CH_3$ | 1 | keine Wirkung |
| 2 | 3-Cl | 0,02 | 100 |
| 3 | 3-Br | 0,001 | 100 |
| 17* | 3-F | 0,002 | 100 |
| 15* | 4-Cl | 0,1 | 100 |
| 27* | 4-Br | 0,4 | 100 |
| | | 0,001 | keine Wirkung |
| 8 | 3,4-$F_2$ | 0,004 | 100 |
| 19* | 2,4-$F_2$ | 0,02 | 100 |

* aus EP-A 258 733

**Patentansprüche**

**1.** Substituierte Cyclopropancarboxamide der allgemeinen Formel I

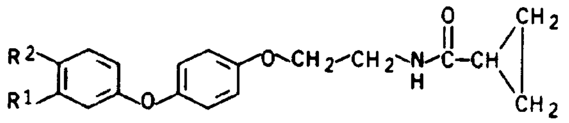

(I),

in der die Substituenten die folgende Bedeutung haben:

$R^1$ $C_1$-$C_4$-Alkyl, Fluor, Chlor oder Brom, $C_1$-$C_3$-Halogenalkyl, $C_1$-$C_3$-Halogenalkoxy

$R^2$ Wasserstoff, Fluor, Chlor oder Brom,

mit der Maßgabe, daß $R^1$ im Fall von $R^2$ = H nicht Fluor bedeutet.

2. Verfahren zur Herstellung von Cyclopropancarboxamiden der allgemeinen Formel I gemäß Anspruch 1, dadurch gekennzeichnet, daß man ein 4-Phenoxyphenoxy-ethylamin der allgemeinen Formel II

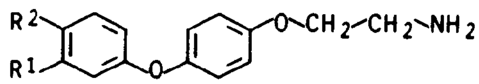

(II)

mit einem Cyclopropancarbonsäurehalogenid der allgemeinen Formel III

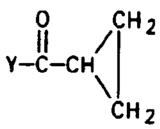

(III)

in der Y für Halogen steht, in Gegenwart eines Säureakzeptors umsetzt.

3. Schädlingsbekämpfungsmittel, enthaltend ein Cyclopropancarboxamid der Formel I gemäß Anspruch 1 neben hierfür üblichen Trägerstoffen.

4. Schädlingsbekämpfungsmittel nach Anspruch 3, dadurch gekennzeichnet, daß es 0,01 bis 95 Gew.% eines Cyclopropancarboxamids der Formel I enthält.

5. Verfahren zur Bekämpfung von Schädlingen dadurch gekennzeichnet, daß man eine wirksame Menge eines Cyclopropancarboxamids der Formel I gemäß Anspruch 1 auf die Schädlinge bzw. deren Lebensraum einwirken läßt.

## Claims

1. Substituted cyclopropanecarboxamides of the general formula I

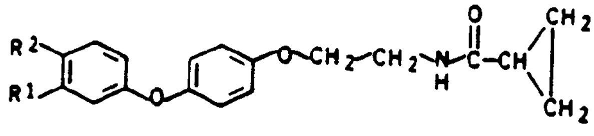

(I),

where $R^1$ is $C_1$-$C_4$-alkyl, fluorine, chlorine, bromine, $C_1$-$C_3$-haloalkyl or $C_1$-$C_3$-haloalkoxy, $R^2$ is hydrogen, fluorine, chlorine or bromine, with the proviso that $R^1$ is not fluorine if $R^2$ is H.

2. A process for preparing cyclopropanecarboxamides of the general formula I as set forth in claim 1, wherein a 4-phenoxyphenoxyethylamine of the general formula II

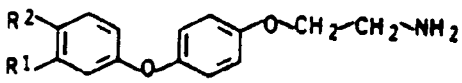

(II)

is reacted with a cyclopropanecarbomyl halide of the general formula III

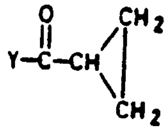

(III)

where Y is halogen, in the presence of an acid acceptor.

3. A pesticide comprising a cyclopropanecarboxamide of the formula I as set forth in claim 1 as well as customary carriers for this purpose.

4. A pesticide as claimed in claim 3, containing from 0.01 to 95 % by weight of a cyclopropanecarboxamide of the formula I.

5. A method for the control of pests, which comprises causing an effective amount of a cyclopropanecarboxamide of the formula I as set forth in claim 1 to act on the pests or their habitat.

## Revendications

1. Cyclopropane-carboxamides substitués de formule générale I

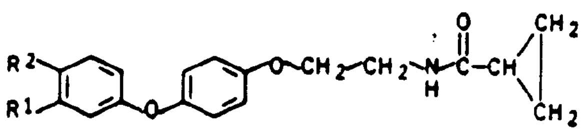

(I),

dans laquelle les symboles ont les significations suivantes :

$R^1$ représente un groupe alkyle en C1-C4, le fluor, le chlore ou le brome, un groupe halogénoalkyle en C1-C3, halogénoalcoxy en C1-C3,

$R^2$ représente l'hydrogène, le fluor, le chlore ou le brome, sous réserve que, lorsque $R^2$ = H, $R^1$ ne peut représenter le fluor.

2. Procédé de préparation des cyclopropane-carboxamides de formule générale I de la revendication 1, caractérisé en ce que l'on fait réagir en présence d'un accepteur d'acide une 4-phénoxyphénoxyéthylamine de formule générale II

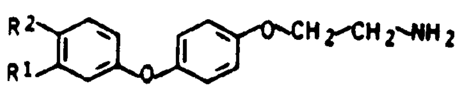

(II)

avec un halogénure d'acide cyclopropane-carboxylique de formule générale III

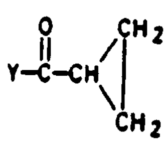

(III)

dans laquelle Y représente un halogène.

3. Produit parasiticide, contenant un cyclopropanecarboxamide de formule I de la revendication 1 avec des véhicules usuels.

4. Produit parasiticide selon la revendication 3, caractérisé en ce qu'il contient de 0,01 à 95 % en poids d'un cyclopropane-carboxamide de formule I.

5. Procédé pour combattre les parasites, caractérisé en ce que l'on fait agir une quantité efficace d'un cyclopropane-carboxamide de formule I de la revendication 1 sur les parasites ou leur habitat.